# EUROPEAN PATENT SPECIFICATION

(11) **EP 2 908 859 B1**
(45) Date of publication and mention of the grant of the patent: **17.05.2017**
(21) Application number: 13783000.6
(22) Date of filing: 11.10.2013
(51) Int. Cl.: A61K 47/02, A61K 47/10, A61K 47/26, A61K 31/496

(54) **ARIPIPRAZOLE FORMULATIONS**
ARIPIPRAZOLFORMULIERUNGEN
FORMULATIONS D'ARIPIPRAZOLE

(30) Priority: 16.10.2012 TR 201211846
(43) Date of publication of application: 26.08.2015
(73) Proprietor: Arven Ilac Sanayi ve Ticaret A.S., Istinye, Istanbul 34460 (TR)
(72) Inventor: TÜRKYILMAZ, Ali, 34460 Istanbul (TR); SUCUOGLU, Esra, 34460 Istanbul (TR)
(74) Representative: Sevinç, Erkan
(86) International application number: PCT/EP2013/071348
(87) International publication number: WO 2014/060324

(56) References cited:
- EP-B1- 1 381 367

## Description

### Field of the Invention

The present invention relates to pharmaceutical formulations of aripiprazole or pharmaceutically acceptable salts thereof. More particularly, the present invention relates to oral solutions of aripiprazole having improved stability, enhanced taste and reduced risk of contamination.

### Background of the Invention

Aripiprazole is an atypical antipsychotic used for the treatment of schizophrenia, bipolar I disorder, major depressive disorder and autistic disorder. Its chemical structure is illustrated with Formula I given below.

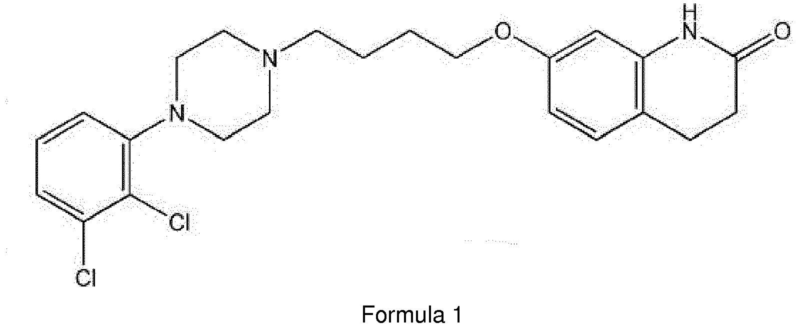

EP0367141 discloses carbostyril derivatives, including aripiprazole specifically.

WO2007113846, WO2006053781 and WO2006030446 disclose processes for obtaining aripiprazole.

Aripiprazole formulations which are in the form of a solution are known from EP 1 381 367 B1. What is disclosed in EP 1 381 367 B1 is aripiprazole solution which comprises a pharmaceutically suitable solvent system, one or more taste-enhancing/masking agents and one or more agents selected from the group consisting of lactic acid, acetic acid, tartaric acid and citric acid, wherein said solution has a pH from 2.5 to 4.5.

Aripiprazole is mostly administrated orally in the form of solid dosage forms. Patients who have difficulties in swallowing the solid forms are in need of oral solution forms of aripiprazole. In addition to this, oral solutions provide flexibility in dosage regimes for patients who need special doses of the drug.

Aripiprazole has stability problems when present in oral solutions experienced with the effect of environmental and physical conditions. Carbostyril compounds in general and aripiprazole specifically are affected from temperature, air and humidity conditions. Exposure to air and humidity causes structural degradation and chemical behaviour changes in the aripiprazole. Thus the stability of the solutions of aripiprazole is not at a desired level and the shelf life thereof is shortened. Additionally, aripiprazole may react with the excipients which are used together in the formulation. This situation leads to impurity in the formulations.

Another disadvantage of aripiprazole is that it has a bitter taste. Thus there is a need to increase the water solubility of aripiprazole and its derivatives and to mask the bitter taste of these compounds so that they can be more incorporated into the aqueous based compositions.

In addition to these disadvantages, oral solutions have the risk of contamination since they contain high amounts of water. Bacteria need moisture to grow and oral solutions contain this moisture. So there is a need to develop an oral solution of aripiprazole that has reduced risk of contamination.

Thus there is still a need for oral solutions of aripiprazole with improved stability, enhanced taste and reduced risk of contamination.

### Summary of the Invention

It is therefore an object of the present invention to provide an oral pharmaceutical solution of aripiprazole or pharmaceutically acceptable salts thereof having improved stability, enhanced taste and reduced risk of contamination.

The inventors of the present invention have discovered that by using hydrochloric acid the solubility of aripiprazole is increased. Using hydrochloric acid also helps to eliminate the risk of contamination. Since it is a strong acid it inhibits the growth of bacteria. When weak acids have been used the risk of contamination is not decreased to the desired levels. On the other hand strong acids may cause irritation to the mucosal surfaces when used in high amounts. Therefore it is another object of the invention to provide an oral pharmaceutical solution of aripiprazole or pharmaceutically acceptable salts thereof having reduced risk of contamination and at the same time does not irritate the mucosal surfaces.

In addition to this the inventors of the present invention have discovered that by using glycerin and ethyl alcohol in the ratio of 4:1 by weight, the solubility of aripiprazole increased and the clearness of the solution is improved. Thus it is another object of the invention to provide an oral pharmaceutical solution of aripiprazole or pharmaceutically acceptable salts thereof comprising hydrochloric acid, glycerin and ethyl alcohol wherein the ratio of glycerin to ethyl alcohol is 4:1.

Another object of the present invention is to provide an oral solution of aripiprazole with enhanced taste. Aripiprazole has a bitter taste and there is a need to mask its taste in an oral solution to increase patient compliance. Flavoring and sweetening agents are used for this purpose.

Further advantages and embodiments of the present invention will become apparent from the following description.

### Detailed Description of Invention

According to the present invention, a novel oral pharmaceutical solution of aripiprazole is obtained surprisingly, which is having improved stability, enhanced taste and reduced risk of contamination. Further, this formulation does not cause irritation to the mucosal surfaces which is an unwanted effect of strong acids in oral solutions.

Aripiprazole useful in accordance with this invention includes the pharmaceutically acceptable salts and esters of aripiprazole, and further includes the conventionally used polymorphic forms of it. The oral pharmaceutical solution of the invention comprises from 0.05 mg/ml to 3 mg/ml aripiprazole, preferably from 0,75 mg/ml to 2 mg/ml, more preferably 1 mg/ml.

The oral pharmaceutical solution of the invention comprises hydrochloric acid from 0.10 mg/ml to 0.60 mg/ml, preferably from 0.15 mg/ml to 0.50 mg/ml, more preferably from 0.20 mg/ml to 0.40 mg/ml. Hydrochloric acid used in this invention is in % 37 concentration.

Hydrochloric acid is a strong acid thus it inhibits the growth of bacteria and as a result of this eliminates the risk of contamination. When weak acids have been used the risk of contamination is not decreased to the desired levels. On the other hand strong acids may cause irritation to the mucosal surfaces when used in high amounts. The present invention provides an oral pharmaceutical solution of aripiprazole or pharmaceutically acceptable salts thereof having reduced risk of contamination and at the same time does not irritate the mucosal surfaces. According to the present invention the solution does not comprise weak acids, such as lactic acid, acetic acid, tartaric acid and citric acid.

The oral pharmaceutical solution of the invention comprises glycerin and ethyl alcohol wherein the weight ratio of glycerin to ethyl alcohol is 4:1. The inventors of present invention have discovered that by using glycerin and ethyl alcohol in this very specific ratio the solubility of aripiprazole increased and the clearness of the solution is improved. The preferred amount of glycerin is from 100 mg/ml to 300 mg/ml, more preferably 150 mg/ml to 250 mg/ml, most preferably 180 mg/ml to 220 mg/ml. The preferred amount of ethyl alcohol is from 25 mg/ml to 75 mg/ml, more preferably 40 mg/ml to 60 mg/ml, most preferably 45 mg/ml to 55 mg/ml.

The oral pharmaceutical solution of the invention further comprises flavoring and sweetening agents to mask the bitter taste of aripiprazole. The sweetening agents are selected from fructose, sucrose, glucose, maltose, sorbitol, mannitol and xylitol. Preferred sweetening agents are fructose and sucrose and the preferred ratio of fructose to sucrose is 1:2 by weight. Preferably fructose is in the amount of between 100 mg/ml to 300 mg/ml, more preferably between 150 mg/ml to 250 mg/ml. Sucrose is preferably in the amount of between 200 mg/ml to 600 mg/ml, more preferably between 300 mg/ml to 500 mg/ml. The flavoring agents are selected from orange, peppermint and cherry. Preferred flavoring agent is orange. The amount of flavoring agent is preferably from 1 mg/ml to 6 mg/ml by weight of the total solution.

The oral pharmaceutical solution according to the present invention comprises preferably on a weight basis:
(i) between 0,75 mg/ml to 2 mg/ml aripiprazole or pharmaceutically acceptable salts thereof;
(ii) between 0.10 mg/ml to 0.60 mg/ml hydrochloric acid;
(iii) between 100 mg/ml to 300 mg/ml glycerin;
(iv) between 25 mg/ml to 75 mg/ml ethyl alcohol;
(v) between 100 mg/ml to 300 mg/ml fructose;
(vi) between 200 mg/ml to 600 mg/ml sucrose;
(vii) between 1 mg/ml to 6 mg/ml orange flavor;

The oral pharmaceutical solution according to the present invention comprises most preferably on a weight basis:
(i) 1 mg/ml aripiprazole or pharmaceutically acceptable salts thereof;
(ii) between 0.20 mg/ml to 0.40 mg/ml hydrochloric acid;
(iii) between 180 mg/ml to 220 mg/ml glycerin;
(iv) between 45 mg/ml to 55 mg/ml ethyl alcohol;
(v) between 150 mg/ml to 250 mg/ml fructose;
(vi) between 300 mg/ml to 500 mg/ml sucrose;
(vii) between 1 mg/ml to 6 mg/ml orange flavor;

The invention is further defined by reference to the following example. Although the example is not intended to limit the scope of the present invention, it should be considered in the light of the description detailed above.

### Example 1

### Preparation of the oral topical aqueous solution

Glycerin and water are mixed in a container. Aripiprazole monohydrate and ethyl alcohol are mixed in another container and they are stirred until they dissolve. Aripiprazole and ethyl alcohol mixture then added to the glycerin and water mixture. They are heated to 50 °C. Fructose and sucrose are added to the mixture and stirred until they dissolve, then cooled down to 30 °C. Orange flavor is added to the mixture. Hydrochloric acid is added. Purified water is added lastly. The mixture is filtered twice.

### Final composition:

| | |
|---|---|
| Aripiprazole | 1 mg/ml |
| Fructose | 200 mg/ml |
| Sucrose | 400 mg/ml |
| Glycerin | 200 mg/ml |
| Orange Flavor | 3 mg/ml |
| Ethyl alcohol | 50 mg/ml |
| Hydrochloric acid (%37) | 0.25 mg/ml |
| Purified water, sufficient for | 1 ml |

## Claims

1. An oral pharmaceutical solution comprising:
(i) aripiprazole or pharmaceutically acceptable salts thereof,
(ii) hydrochloric acid,
(iii) glycerin and ethyl alcohol
wherein the weight ratio of glycerin to ethyl alcohol is 4:1 and the hydrochloric acid is in an amount of 0.10 mg/ml to 0.60 mg/ml.

2. The solution according to claims 1 wherein the glycerin is in an amount of 100 mg/ml to 300 mg/ml.

3. The solution according to claim 1 and 2 comprising on a weight basis,
(i) between 0,75 mg/ml to 2 mg/ml aripiprazole or pharmaceutically acceptable salts thereof;
(ii) between 0.10 mg/ml to 0.60 mg/ml hydrochloric acid;
(iii) between 100 mg/ml to 300 mg/ml glycerin;
(iv) between 45 mg/ml to 55 mg/ml ethyl alcohol;

4. The solution according to claims 1 to 3 further comprising at least two sweetening agents selected from fructose, sucrose, glucose, maltose, sorbitol, mannitol and xylitol.

5. The solution according to claim 4 wherein the sweeting agents are fructose and sucrose.

6. The solution according to claims 4 and 5 wherein the ratio of fructose to sucrose is 1:2 by weight.

7. The solution according to claims 1 to 6 comprising on a weight basis,
(i) between 0,75 mg/ml to 2 mg/ml aripiprazole or pharmaceutically acceptable salts thereof;
(ii) between 0.10 mg/ml to 0.60 mg/ml hydrochloric acid;
(iii) between 100 mg/ml to 300 mg/ml glycerin;
(iv) between 25 mg/ml to 75 mg/ml ethyl alcohol;
(v) between 100 mg/ml to 300 mg/ml fructose;
(vi) between 200 mg/ml to 600 mg/ml sucrose;
(vii) between 1 mg/ml to 6 mg/ml orange flavor;

8. The solution according to claims 1 to 7, further comprising at least one flavoring agent in an amount of from 1 mg/ml to 6 mg/ml by weight of the total solution.

9. The solution according to any of the preceding claims, comprising on a weight basis:
(i) 1 mg/ml aripiprazole or pharmaceutically acceptable salts thereof;
(ii) between 0.20 mg/ml to 0.40 mg/ml hydrochloric acid;
(iii) between 180 mg/ml to 220 mg/ml glycerin;
(iv) between 45 mg/ml to 55 mg/ml ethyl alcohol;
(v) between 150 mg/ml to 250 mg/ml fructose;
(vi) between 300 mg/ml to 500 mg/ml sucrose;
(vii) between 1 mg/ml to 6 mg/ml orange flavor;

## Patentansprüche

1. Orale pharmazeutische Lösung, umfassend:
(i) Aripiprazol oder pharmazeutisch akzeptable Salze davon,
(ii) Salzsäure,
(iii) Glycerin und Ethylalkohol,
wobei das Gewichtsverhältnis von Glycerin zu Ethylalkohol 4:1 ist, und die Salzsäure in einer Menge von 0,10 mg/ml bis 0,60 mg/ml vorliegt.

2. Lösung nach Anspruch 1, wobei das Glycerin in einer Menge von 100 mg/ml bis 300 mg/ml vorliegt.

3. Lösung nach Anspruch 1 und 2, umfassend, auf einer Gewichtsbasis,
(i) zwischen 0,75 mg/ml bis 2 mg/ml Aripiprazol oder pharmazeutisch akzeptable Salze davon;
(ii) zwischen 0.10 mg/ml bis 0.60 mg/ml Salzsäure;
(iii) zwischen 100 mg/ml bis 300 mg/ml Glycerin;
(iv) zwischen 45 mg/ml bis 55 mg/ml Ethylalkohol.

4. Lösung nach Ansprüchen 1 bis 3, weiterhin umfassend wenigstens zwei Süßstoffe, ausgewählt aus Fructose, Saccharose, Glucose, Maltose, Sorbitol, Mannitol und Xylitol.

5. Lösung nach Anspruch 4, wobei die Süßstoffe Fructose und Saccharose sind.

6. Lösung nach Ansprüchen 4 und 5, wobei das Verhältnis von Fructose zur Saccharose 1:2 beträgt, bezogen auf das Gewicht.

7. Lösung nach Ansprüchen 1 bis 6, umfassend, auf einer Gewichtsbasis,
(i) zwischen 0,75 mg/ml bis 2 mg/ml Aripiprazol oder pharmazeutisch akzeptable Salze davon;
(ii) zwischen 0.10 mg/ml bis 0.60 mg/ml Salzsäure;
(iii) zwischen 100 mg/ml bis 300 mg/ml Glycerin;
(iv) zwischen 25 mg/ml bis 75 mg/ml Ethylalkohol;
(v) zwischen 100 mg/ml bis 300 mg/ml Fructose;
(vi) zwischen 200 mg/ml bis 600 mg/ml Saccharose;
(vii) zwischen 1 mg/ml bis 6 mg/ml Orangengeschmack.

8. Lösung nach Ansprüchen 1 bis 7, weiterhin umfassend wenigstens einen Aromastoff in einer Menge von 1 mg/ml bis 6 mg/ml, bezogen auf das Gewicht der Gesamtlösung.

9. Lösung nach einem der vorangehenden Ansprüche, umfassend, auf einer Gewichtsbasis:
(i) 1 mg/ml Aripiprazol oder pharmazeutisch akzeptable Salze davon;
(ii) zwischen 0.20 mg/ml bis 0.40 mg/ml Salzsäure;
(iii) zwischen 180 mg/ml bis 220 mg/ml Glycerin;
(iv) zwischen 45 mg/ml bis 55 mg/ml Ethylalkohol;
(v) zwischen 150 mg/ml bis 250 mg/ml Fructose;
(vi) zwischen 300 mg/ml bis 500 mg/ml Saccharose;
(vii) zwischen 1 mg/ml bis 6 mg/ml Orangengeschmack.

## Revendications

1. Solution pharmaceutique orale, comprenant :
(i) de l'aripiprazole ou ses sels pharmaceutiquement acceptables,
(ii) de l'acide chlorhydrique,
(ii) de la glycérine et de l'alcool éthylique
dans laquelle le rapport en poids de la glycérine à l'alcool éthylique est de 4:1 et l'acide chlorhydrique est en une quantité de 0,10 mg/ml à 0,60 mg/ml.

2. Solution selon la revendication 1, dans laquelle la glycérine est en une quantité de 100 mg/ml à 300 mg/ml.

3. Solution selon les revendications 1 et 2, comprenant sur une base en poids,
(i) entre 0,75 mg/ml à 2 mg/ml d'aripiprazole ou de ses sels pharmaceutiquement acceptables ;
(ii) entre 0,10 mg/ml à 0,60 mg/ml d'acide chlorhydrique ;
(iii) entre 100 mg/ml à 300 mg/ml de glycérine ;
(iv) entre 45 mg/ml à 55 mg/ml d'alcool éthylique.

4. Solution selon les revendications 1 à 3, comprenant en outre au moins deux agents édulcorants choisis parmi le fructose, le saccharose, le glucose, le maltose, le sorbitol, le mannitol et le xylitol.

5. Solution selon la revendication 4, dans laquelle les agents édulcorants sont le fructose et le saccharose.

6. Solution selon les revendications 4 et 5, dans laquelle le rapport du fructose au saccharose est de 1:2 en poids.

7. Solution selon les revendications 1 à 6, comprenant sur une base en poids :
(i) entre 0,75 mg/ml à 2 mg/ml d'aripiprazole ou de ses sels pharmaceutiquement acceptables ;
(ii) entre 0,10 mg/ml à 0,60 mg/ml d'acide chlorhydrique ;
(iii) entre 100 mg/ml à 300 mg/ml de glycérine ;
(iv) entre 25 mg/ml à 75 mg/ml d'alcool éthylique ;
(v) entre 100 mg/ml à 300 mg/ml de fructose ;
(vi) entre 200 mg/ml à 600 mg/ml de saccharose ;
(vii) entre 1 mg/ml à 6 mg/ml de saveur d'orange.

8. Solution selon les revendications 1 à 7, comprenant en outre au moins un agent aromatisant en une quantité de 1 mg/ml à 6 mg/ml en poids de la solution totale.

9. Solution selon l'une quelconque des revendications précédentes, comprenant sur une base en poids :
(i) 1 mg/ml d'aripiprazole ou de ses sels pharmaceutiquement acceptables ;
(ii) entre 0,20 mg/ml à 0,40 mg/ml d'acide chlorhydrique ;
(iii) entre 180 mg/ml à 220 mg/ml de glycérine ;
(iv) entre 45 mg/ml à 55 mg/ml d'alcool éthylique ;
(v) entre 150 mg/ml à 250 mg/ml de fructose ;
(vi) entre 300 mg/ml à 500 mg/ml de saccharose ;
(vii) entre 1 mg/ml à 6 mg/ml de saveur d'orange.
